# EUROPÄISCHE PATENTSCHRIFT

(11) **EP 1 886 702 B1**
(45) Veröffentlichungstag und Bekanntmachung des Hinweises auf die Patenterteilung: **11.10.2017**
(21) Anmeldenummer: 07013442.4
(22) Anmeldetag: 10.07.2007
(51) Int. Cl.: A61L 27/04, A61L 27/28, A61L 31/02, C07F 7/08, A61L 27/34, A61L 31/10, A61L 31/08

(54) **Implantat aus einem biokorrodierbaren metallischen Werkstoff mit einer Beschichtung aus einer Organosiliziumverbindung**
Implant made of a biocorrodible metal substance with a coating of an organo-silicon compound
Implant en matière première métallique biocorrodable doté d'un revêtement en alliage d'organosilicium

(30) Priorität: 07.08.2006 DE 102006038231
(43) Veröffentlichungstag der Anmeldung: 13.02.2008
(73) Patentinhaber: Biotronik VI Patent AG, 6341 Baar (CH)
(72) Erfinder: Borck, Alexander, Dr., 91086 Aurachtal (DE); Rzany, Alexander, Dr., 90449 Nürnberg (DE); Wittchow, Eric, Dr., 90403 Nürnberg (DE)
(74) Vertreter: Randoll, Sören

(56) Entgegenhaltungen:
- EP-A2- 0 664 452
- WO-A1-03/026717
- US-A1- 2004 236 399

## Beschreibung

Die Erfindung betrifft ein Implantat aus einem biokorrodierbaren metallischen Werkstoff mit einer Beschichtung aus einer Siliziumverbindung sowie ein dazugehöriges Verfahren zur Herstellung des Implantats.
Medizinische Implantate unterschiedlichster Zweckbestimmung sind in großer Vielfalt aus dem Stand der Technik bekannt. Häufig ist nur ein zeitweiliger Verbleib des Implantats im Körper zur Erfüllung des medizinischen Zweckes erforderlich. Implantate aus permanenten Werkstoffen, also Werkstoffen, die im Körper nicht abgebaut werden, sind wieder zu entfernen, da es mittel- und langfristig auch bei hoher Biokompatibilität zu Abstoßungsreaktionen des Körpers kommen kann.
Ein Ansatz zur Vermeidung eines weiteren chirurgischen Eingriffs besteht nun darin, das Implantat ganz oder in Teilen aus einem biokorrodierbaren Werkstoff zu formen. Unter Biokorrosion werden mikrobielle Vorgänge oder schlicht durch die Anwesenheit von Körpermedien bedingte Prozesse verstanden, die zu einem allmählichen Abbau der aus dem Werkstoff bestehen Struktur führen. Zu einem bestimmten Zeitpunkt verliert das Implantat oder zumindest der Teil des Implantates, der aus dem biokorrodierbaren Werkstoff besteht, seine mechanische Integrität. Die Abbauprodukte werden vom Körper weitgehend resorbiert. Diese können, wie beispielsweise Magnesium, lokal sogar therapeutische Wirkung entfalten. Geringe Mengen nicht resorbierbarer Legierungsbestandteile sind tolerierbar.
Biokorrodierbare Werkstoffe wurden unter anderem auf Basis von Polymeren synthetischer Natur oder natürlichem Ursprungs entwickelt. Die mechanischen Materialeigenschaften (geringe Plastizität), aber auch teils die geringe Biokompatibilität der Abbauprodukte der Polymere (teils erhöhte Thrombogenität, vermehrte Inflammation), limitiert den Einsatz jedoch deutlich. So müssen beispielsweise orthopädische Implantate häufig hohen mechanischen Beanspruchungen standhalten und vaskuläre Implantate, z.B. Stents, je nach Design sehr speziellen Anforderungen an E-Modul, Brüchigkeit und Formbarkeit genügen.

Ein aussichtsreicher Ansatz zur Lösung des Problems liegt in der Verwendung biokorrodierbarer Metalllegierungen. So wird in DE 197 31 021 A1 vorgeschlagen, medizinische Implantate aus einem metallischen Werkstoff zu formen, dessen Hauptbestandteil ein Element aus der Gruppe Alkalimetalle, Erdalkalimetalle, Eisen, Zink und Aluminium ist. Als besonders geeignet werden Legierungen auf Basis von Magnesium, Eisen und Zink beschrieben. Nebenbestandteile der Legierungen können Mangan, Cobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90%, Yttrium 3,7 - 5,5%, Seltenerdmetallen 1,5 - 4,4% und Rest < 1% bekannt, die sich insbesondere zur Herstellung einer Endoprothese, z. B. in Form eines selbstexpandierenden oder ballonexpandierbaren Stent, eignet. Ungeachtet der erreichten Fortschritte auf dem Gebiet biokorrodierbarer Metalllegierungen sind die bisher bekannten Legierungen aufgrund ihres Korrosionsverhaltens nur beschränkt einsatzfähig. Die relativ rasche Biokorrosion der Magnesiumlegierungen, insbesondere im Bereich mechanisch stark belasteter Strukturen, limitiert den Einsatz.

Sowohl die Grundlagen der Magnesium-Korrosion als auch eine große Anzahl von technischen Verfahren zur Verbesserung des Korrosionsverhaltens (im Sinne einer Verstärkung des Korrosionsschutzes) sind aus dem Stand der Technik bekannt. Bekannt ist beispielsweise, dass der Zusatz von Yttrium und/oder weiteren Seltenerdmetallen einer Magnesiumlegierung einen leicht erhöhten Korrosionswiderstand in Meerwasser verleiht.

Ein Ansatzpunkt sieht vor, eine korrosionsschützende Schicht auf dem aus Magnesium oder einer Magnesiumlegierung bestehenden Formkörper zu erzeugen. Bekannte Verfahren zur Erzeugung einer korrosionsschützenden Schicht wurden unter dem Gesichtpunkt eines technischen Einsatzes des Formkörpers - jedoch nicht medizin-technischen Einsatzes in biokorrodierbaren Implantaten in physiologischer Umgebung - entwickelt und optimiert. Diese bekannten Verfahren umfassen: das Aufbringen von Polymeren oder anorganischen Deckschichten, das Erzeugen einer Emaille, die chemische Konversion der Oberfläche, Heißgasoxidation, Anodisieren, Plasmaspritzen, Laserstrahl-Umschmelzen, PVD-Verfahren, lonenimplantation oder Lackieren.
Herkömmliche technische Einsatzgebiete von Formkörpern aus Magnesiumlegierungen außerhalb der Medizintechnik erfordern in der Regel eine weitgehende Unterbindung korrosiver Prozesse. Dementsprechend ist die Zielstellung der meisten technischen Verfahren eine vollständige Inhibierung korrosiver Prozesse. Dagegen sollte die Zielstellung zur Verbesserung des Korrosionsverhaltens von biokorrodierbaren Magnesiumlegierungen nicht in der vollständigen Unterbindung, sondern nur in der Hemmung korrosiver Prozesse liegen. Schon aus diesem Grunde eignen sich die meisten bekannten Verfahren zur Erzeugung einer Korrosionsschutzschicht nicht. Ferner müssen für einen medizin-technischen Einsatz auch toxikologische Aspekte berücksichtigt werden. Des Weiteren sind korrosive Prozesse stark von dem Medium abhängig, in dem sie ablaufen, und daher dürfte eine Übertragbarkeit der unter herkömmlichen Umweltbedingungen auf technischem Gebiet gewonnenen Erkenntnisse zum Korrosionsschutz auf die Prozesse in physiologischer Umgebung nicht uneingeschränkt möglich sein. Schließlich dürften bei einer Vielzahl von medizinischen Implantaten auch die der Korrosion zugrunde liegenden Mechanismen von üblichen technischen Anwendungen des Werkstoffs abweichen. So werden beispielsweise Stents, chirurgisches Nahtmaterial oder Clips im Gebrauch mechanisch verformt, so dass der Teilprozess der Spannungsriss-Korrosion beim Abbau dieser Formkörper erhebliche Bedeutung haben dürfte.
Zusätzlich ist zu beachten, dass bei Implantaten wie Stents lokal hohe plastische Verformungen des Grundkörpers auftreten. Konventionelle Verfahren wie die Generierung einer dichten Magnesiumoxid-Schicht, welche auch OH-Gruppen enthalten kann, sind für diese Anwendung nicht zielführend. Die keramischen Eigenschaften der Deckschicht würden zu einem Iokalen Abplatzen bzw. einer Rissbildung führen. Die Korrosion wäre damit in unkontrollierter Weise im Bereich der eigentlich besonders zu schützenden mechanisch belasteten Stellen lokal fokussiert.

WO 03/026717 offenbart ein medizinisches Implantat wie z.B. ein Stent, welches aus einer Magnesiumlegierung besteht. Die Organosiliziumverbindung, die für die Beschichtung verwendet wird, kann n-Octadecyltriethoxysilan, 3-Mercaptopropyltrimethoxysilan oder 3-Aminopropyltriethoxysilan seine.

DE 101 63 106 A1 sieht vor, den Magnesiumwerkstoff durch Modifikation mit Halogeniden in seiner Korrosivität zu verändern. Der Magnesiumwerkstoff soll zur Herstellung medizinischer Implantate verwendet werden. Bevorzugt ist das Halogenid ein Fluorid. Die Modifikation des Werkstoffs wird durch Zulegieren salzförmiger Halogenverbindungen erreicht. Es wird demnach die Zusammensetzung der Magnesiumlegierung durch Zugabe der Halogenide verändert, um die Korrosionsrate zu mindern. Dementsprechend wird der gesamte, aus einer solchen modifizierten Legierung bestehende Formkörper ein verändertes Korrosionsverhalten besitzen. Durch das Zulegieren können jedoch weitere Werkstoffeigenschaften beeinflusst werden, die bei der Verarbeitung von Bedeutung sind oder auch die mechanischen Eigenschaften des aus dem Werkstoff entstehenden Formkörpers prägen.
Ferner sind Beschichtungen für Implantate aus nicht-biokorrodierbaren also permanenten Werkstoffen bekannt, die auf Organosiliziumverbindungen basieren. So beschreibt beispielsweise DE 699 12 951 T2 eine Zwischenschicht aus einem funktionalisierten Silikonpolymer, wie Siloxanen oder Polysilanen. US 2004/0236399 A1 offenbart einen Stent mit einer Silanschicht, die von einer weiteren Schicht bedeckt wird.
Der vorliegenden Erfindung liegt die Aufgabe zugrunde, eine alternative oder verbesserte Beschichtung für ein Implantat aus einem biokorrodierbaren Werkstoff bereitzustellen, die eine temporäre Inhibition, aber nicht vollständige Unterbindung der Korrosion des Werkstoffs in physiologischer Umgebung bewirkt.
Diese Aufgabe wird nach einem ersten Aspekt der Erfindung durch das erfindungsgemäße Implantat aus einem biokorrodierbaren metallischen Werkstoff gelöst. Dazu ist vorgesehen, dass das Implantat eine Beschichtung aus einer Organosiliziumverbindung der Formel (1) aufweist: 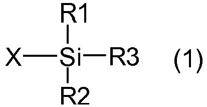 wobei X für eine O-, S- oder N-Funktionalität an einer Oberfläche des Implantats steht, über die eine kovalente Anbindung der Organosiliziumverbindung an die Oberfläche des Implantats erfolgt;
R1 und R2 unabhängig voneinander festgelegt ein substituierter oder unsubstituierter Alkylrest mit 1 bis 5 C-Atomen oder eine Sauerstoffbrücke zu einer benachbarten Organosiliziumverbindung ist; und R3 ein substituierter oder unsubstituierter Alkylrest ist und der Alkylrest 3 bis 30 C-Atomen aufweist, wobei als Substituenten Halogenide, Aromaten oder heteroaromatische Verbindungen vorhanden sind, bei dem der biokorrodierbare metallische Werkstoff eine biokorrodierbare Legierung ausgewählt aus der Gruppe Magnesium, Eisen und Wolfram ist. Es hat sich gezeigt, dass die Aufbringung einer Beschichtung der genannten Zusammensetzung nicht zur Ausbildung einer vollständig oder weitgehend die Korrosion in physiologischer Umgebung inhibierenden Schutzschicht führt. Mit anderen Worten, in physiologischer Umgebung erfolgt dennoch eine Korrosion des Implantats, jedoch mit deutlich verzögerter Geschwindigkeit. Der biokorrodierbare metallische Werkstoff ist eine biokorrodierbare Legierung ausgewählt aus der Gruppe der Elemente Magnesium, Eisen und Wolfram; insbesondere ist der Werkstoff eine biokorrodierbare Magnesiumlegierung. Unter Legierung wird vorliegend ein metallisches Gefüge verstanden, deren Hauptkomponente Magnesium, Eisen oder Wolfram ist. Hauptkomponente ist die Legierungskomponente, deren Gewichtsanteil an der Legierung am höchsten ist. Ein Anteil der Hauptkomponente beträgt vorzugsweise mehr 50 Gew.%, insbesondere mehr als 70 Gew.%.
Ist der Werkstoff eine Magnesiumlegierung, so enthält diese vorzugsweise Yttrium und weitere Seltenerdmetalle, da sich eine derartige Legierung aufgrund ihrer physikochemischen Eigenschaften und hohen Biokompatibilität, insbesondere auch seiner Abbauprodukte, auszeichnet.
Besonders bevorzugt wird eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden.

Die Legierungen der Elemente Magnesium, Eisen oder Wolfram sind so in ihrer Zusammensetzung zu wählen, dass sie biokorrodierbar sind. Als biokorrodierbar im Sinne der Erfindung werden Legierungen bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/I). Eine Probe der zu untersuchenden Legierung wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Der Begriff Korrosion bezieht sich vorliegend auf die Reaktion eines metallischen Werkstoffes mit seiner Umgebung, wobei eine messbare Veränderung des Werkstoffs bewirkt wird, die - bei Einsatz des Werkstoffs in einem Bauteil - zu einer Beeinträchtigung der Funktion des Bauteils führt. Ein Korrosionssystem besteht vorliegend aus dem korrodierenden metallischen Werkstoff sowie einem flüssigen Korrosionsmedium, das in seiner Zusammensetzung die Verhältnisse in physiologischer Umgebung nachstellt oder ein physiologisches Medium, insbesondere Blut ist. Werkstoffseitig beeinflussen die Korrosion Faktoren, wie die Zusammensetzung und Vorbehandlung der Legierung, mikro- und submikroskopische Inhomogenitäten, Randzoneneigenschaften, Temperatur- und mechanischer Spannungszustand und insbesondere die Zusammensetzung einer die Oberfläche bedeckenden Schicht. Auf Seiten des Mediums wird der Korrosionsprozess durch Leitfähigkeit, Temperatur, Temperaturgradienten, Azidität, Volumen-Oberflächen-Verhältnis, Konzentrationsunterschied sowie Strömungsgeschwindigkeit beeinflusst.

An der Phasengrenzfläche zwischen Werkstoff und Medium laufen Redox-Reaktionen ab. Für eine schützende bzw. hemmende Wirkung müssen vorhandene Schutzschichten und/oder die Produkte der Redox-Reaktionen eine gegen das Korrosionsmedium ausreichend dichte Struktur ausbilden, eine bezogen auf die Umgebung erhöhte thermodynamische Stabilität aufweisen und im Korrosionsmedium wenig löslich oder unlöslich sein. In der Phasengrenzfläche, genauer in einer sich in diesem Bereich ausbildenden Doppelschicht, laufen Ad- und Desorptionsprozesse ab. Die Vorgänge in der Doppelschicht sind geprägt von den dort ablaufenden kathodischen, anodischen und chemischen Teilprozessen. Bei Magnesiumlegierungen ist in der Regel eine allmähliche Alkalisierung der Doppelschicht zu beobachten. Fremdstoffablagerungen, Verunreinigungen und Korrosionsprodukte beeinflussen den Korrosionsprozess. Die Vorgänge bei der Korrosion sind demnach hoch komplex und lassen sich gerade im Zusammenhang mit einem physiologischen Korrosionsmedium, also Blut oder künstlichem Plasma, nicht oder nur im geringen Umfang voraussagen, da Vergleichsdaten fehlen. Schon aus diesem Grunde ist das Auffinden eine einer korrosionshemmenden Beschichtung, d. h. einer Beschichtung, die nur zur temporären Herabsetzung der Korrosionsrate eines metallischen Werkstoffs der weiter oben genannten Zusammensetzung in physiologischer Umgebung dient, eine außerhalb der Routine eines Fachmanns liegende Maßnahme. Dies gilt in besonderem Maße für Stents, welche zum Zeitpunkt der Implantation lokal hohen plastischen Verformungen ausgesetzt sind. Konventionelle Ansätze mit starren korrosionshemmenden Schichten sind für derartige Voraussetzungen ungeeignet.

Der Vorgang der Korrosion lässt sich durch Angabe einer Korrosionsrate quantifizieren. Ein zügiger Abbau ist mit einer hohen Korrosionsrate verbunden, und umgekehrt. Bezogen auf den Abbau des gesamten Formkörpers wird eine im Sinne der Erfindung modifizierte Oberfläche zur Herabsetzung der Korrosionsrate führen. Die erfindungsgemäße korrosionshemmende Beschichtung kann im Laufe der Zeit selbst abgebaut werden bzw. kann die von ihr abgedeckten Bereiche des Implantats nur noch in einem immer geringeren Umfang schützen. Daher ist der Verlauf der Korrosionsrate für das gesamte Implantat nicht linear. Vielmehr ergibt sich zu Beginn der einsetzenden korrosiven Prozesse eine relativ niedrige Korrosionsrate, die im Laufe der Zeit ansteigt. Dieses Verhalten wird als temporäre Herabsetzung der Korrosionsrate im Sinne der Erfindung verstanden und zeichnet die korrosionshemmende Beschichtung aus. Im Fall von Koronarstents sollte die mechanische Integrität der Struktur über ein Zeitraum von drei Monaten nach Implantation aufrechterhalten werden.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defibrillatoren. Das Implantat besteht ganz oder in Teilen aus dem biokorrodierbaren Werkstoff. Wenn das Implantat nur in Teilen aus dem biokorrodierbaren Werkstoff besteht, so ist dieser Teil entsprechend zu beschichten.

Vorzugsweise ist das Implantat ein Stent. Stents herkömmlicher Bauart weisen eine filigrane Struktur aus metallischen Streben auf, die zur Einbringung in den Körper zunächst in einem nicht-expandierten Zustand vorliegt und die am Ort der Applikation dann in einen expandierten Zustand aufgeweitet wird. Bei Stents bestehen besondere Anforderungen an die korrosionshemmende Schicht: Die mechanische Belastung des Materials während der Expansion des Implantats hat Einfluss auf den Verlauf des Korrosionsprozesses und es ist davon auszugehen, dass die Spannungsrisskorrosion in den belasteten Bereichen verstärkt wird. Eine korrosionshemmende Schicht sollte diesen Umstand berücksichtigen. Weiterhin könnte eine harte korrosionshemmende Schicht während der Expansion des Stents abplatzen und eine Rissbildung in der Schicht bei Expansion des Implantats dürfte unvermeidbar sein. Schließlich sind die Dimensionen der filigranen metallischen Struktur zu beachten und es sollte nach Möglichkeit nur eine dünne, aber auch gleichmäßige korrosionshemmende Schicht erzeugt werden. Es hat sich nun gezeigt, dass das Aufbringen der erfindungsgemäßen Beschichtung ganz oder zumindest weitgehend diesen Anforderungen genügt.

Die zur Anbindung der Organosiliziumverbindung der Formel (1) notwendige Funktionalität auf der Oberfläche des Implantats kann durch eine gezielte Vorbehandlung an der Oberfläche bereitgestellt werden. So kann beispielsweise eine Plasmabehandlung in sauerstoff- oder stickstoffreicher Atmosphäre dem weiteren Schritten bei der Herstellung der Beschichtung vorgeschaltet werden.

Die Reste R1 und R2 können weitere Substituenten tragen, z. B. Halogenide, insbesondere Chlor. Vorzugsweise sind die Reste R1 und R2 jedoch unsubstituiert und entsprechen einem Substituenten ausgewählt aus der Gruppe Methyl, Ethyl, n-Propyl und i-Propyl. Wenn R1 oder R2 eine Sauerstoffbrücke ist, so bindet dieser gemeinsame Substituent zwei Organosiliziumverbindungen der Formel (1) aneinander. Sind R1 und R2 jeweils eine Sauerstoffbrücke, so bildet sich ein polymeres Netzwerk aus Organosiliziumverbindungen der Formel (1).

R3 ist ein substituierter Alkyl- oder Heteroalkylrest mit 3 bis 30 C-Atomen. Als Substituenten kann beispielsweise Chlorvorhanden sein. Besonders bevorzugt ist es, wenn R3 terminal, also an seinem vom Silizium abgewandten Kettenende, einen reaktiven Substituenten trägt. Dieser reaktive Substituent kann beispielsweise eine Alkoholgruppe, Säuregruppe, eine Vinylverbindung, Isocyanat gekappte Urethane, ein Epoxid oder ein Amin sein. Durch Umsetzung mit geeigneten Substraten kann der reaktive Substituent beispielsweise zur Anbindung von pharmazeutisch aktiven Wirkstoffen oder Biomolekülen (z. B. Oligonucleotide und Enzyme) oder zur Fixierung weiterer Beschichtungen dienen (z. B. Kopplung mit wasserlöslichen Carbodiimide).
Weiterhin ist R3 vorzugsweise ein substituierter Alkylrest mit 5 bis 15 C-Atomen, wobei 1 bis 3 C-Atome durch ein Heteroatom, ausgewählt aus der Gruppe O, N und S, ersetzbar sind. Der Substituent R3 ist ferner vorzugsweise unverzweigt. Der Substituent kann auch aus der Gruppe substituierter oder unsubstituierter aromatischer oder heteroaromatischer Verbindungen stammen, die über eine vorzugsweise unverzweigt Alkylkette von 1-5 Kohlenstoffatomen mit dem Siliziumatom verbunden sind. Schließlich ist R3 bevorzugt ein Rest ausgewählt aus der Gruppe 3-Mercapto-Propyl, n-Propyl, n-Hexyl, n-Octyl, n-Decyl, n-Tetradecyl, n-Octadecyl, 3-Aminopropyl, N-(2-Aminoethyl)-3-Aminopropyl, N-(6-Aminohexyl)-Aminopropyl.
Ein zweiter Aspekt der Erfindung bezieht sich auf ein Verfahren zur Herstellung eines Implantats aus einem biokorrodierbaren metallischen Werkstoff, dessen Oberfläche von einer Beschichtung aus einer Organosiliziumverbindung der vorgenannten Ausprägung bedeckt ist. Das erfindungsgemäße Verfahren umfasst die Schritte:
(i) Bereitstellen eines Rohlings für das Implantats aus dem biokorrodierbaren metallischen Werkstoff;
(ii) Vorbehandeln einer Rohlingsoberfläche zur Erzeugung von O-, S- oder N-Funktionalitäten durch Plasmabehandlung in Sauerstoff- oder stickstoffreicher Atmosphäre; und
(iii) Beschichten der Rohlingsoberfläche mit einem Organosiliziumreagenz, das unter Ausbildung einer kovalenten Bindung zwischen Silizium und einer O-, S- oder N-Funktionalität abreagiert, wobei sich entweder die Organosiliziumverbindung der folgenden Formel direkt bildet oder zunächst eine Vorläufer-Organosiliziumverbindung anfällt, die über weitere Behandlungsschritte in die Organosiliziumverbindung der folgenden Formel überführt wird: 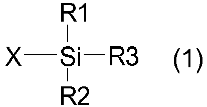 wobei X für eine O-, S- oder N-Funktionalität an einer Oberfläche des Implantats steht, über die eine kovalente Anbindung der Organosiliziumverbindung an die Oberfläche des Implantats erfolgt;
   R1 und R2 unabhängig voneinander festgelegt ein substituierter oder unsubstituierter Alkylrest mit 1 bis 5 C-Atomen oder eine Sauerstoffbrücke zu einer benachbarten Organosiliziumverbindung ist; und
   R3 ein substituierter oder unsubstituierter Alkylrest oder eine Alkylbrücke zu einer benachbarten Organosiliziumverbindung ist und der Alkylrest/die Alkylbrücke 3 bis 30 C-Atomen aufweist, wobei 1, 2 oder 3 C-Atome durch ein Heteroatom ausgewählt aus der Gruppe O, S und N ersetzbar sind.

Mit Hilfe des Verfahrens lassen sich demnach auch die erfindungsgemäßen Beschichtungen aus einer Organosiliziumverbindung der Formel (1) auf der Implantatsoberfläche erzeugen.

Im Schritt (i) des Verfahrens wird ein Rohling für das Implantat bereitgestellt, z. B. in Form eines metallischen Grundkörpers für einen Stent.

Im Schritt (ii) des Verfahrens kann die Rohlingsoberfläche vorbehandelt werden, um die für die Anbindung der Organosiliziumverbindung notwendige Funktionalität auf der Oberfläche des Implantates zu etablieren. Dies geschieht durch Behandlung mit sauerstoff- oder stickstoffreichem Plasma, wobei OH- und NH-Funktionalitäten auf der Oberfläche nach der Behandlung bereitstehen. Bei entsprechend reaktiven Materialien können OH-Gruppen auch durch Tauchen in Wasser, Basen oder Säuren generiert werden.

Im Schritt (iii) des Verfahrens wird die Rohlingsoberfläche mit einem Organosiliziumreagenz beschichtet. Dieser Arbeitsschritt umfasst beispielsweise das Besprühen der Rohlingsoberfläche mit dem Reagenz oder einer Lösung des Reagenzes in einem geeigneten Lösungsmittel mit definiertem Wassergehalt.

Das Organosiliziumreagenz weist eine geeignete Abgangsgruppe auf, die unter Ausbildung einer kovalenten Bindung zwischen Silizium und einer der O-, S- oder N-Funktionalitäten auf der Oberfläche des Implantates substituiert wird. Die Fluchtgruppe ist vorzugsweise Chlor, eine Methoxygruppe, oder eine Ethoxygruppe. Das Organosiliziumreagenz trägt weiterhin entweder bereits die identischen Reste R1 bis R3 der herzustellenden Organosiliziumverbindung der Formel (1) oder es bildet sich zunächst nur eine Zwischenstufe, d. h. eine Vorläufer- Organosiliziumverbindung fällt an. Diese wird dann durch weitere Behandlungsschritte in die gewünschte Organosiliziumverbindung der Formel (1) überführt.

Beispiele für die zweite Verfahrensvariante über eine Vorläufer-Organosiliziumverbindung umfassen insbesondere Organosiliziumverbindungen der Formel (1), bei denen R1 und/oder R2 eine Sauerstoffbrücke zu einer benachbarten Organosiliziumverbindung bilden (entspricht einer Polysiloxanbeschichtung). Das Organosiliziumreagenz weist - neben dem Rest R3 - ein oder zwei Fluchtgruppen auf, die später die Sauerstoffbrücke der Reste R1 bzw. R2 bilden. Diese Fluchtgruppen können z. B. Halogenide oder eine Methoxygruppe umfassen. Nach der Anbindung an die Oberfläche des Implantats erfolgt eine Vernetzung in wässrigen alkalischer Umgebung zur gewünschten Organosiliziumverbindung der Formel (1). Optional kann das Werkstück anschließend durch Kohlendioxid an Luft innerhalb mehrerer Stunden neutralisiert werden

Die Erfindung wird nachfolgend anhand von Ausführungsbeispielen und den dazugehörigen Zeichnungen näher erläutert. Es zeigen:
- Fig. 1: eine schematische Darstellung zur Illustration der Vorgänge bei einer Beschichtung der Implantatsoberfläche;
- Fig. 2: eine schematische Darstellung einer Beschichtung, bei der die Organosiliziumverbindung terminal einen reaktiven Substituenten trägt; und
- Fig. 3: eine schematische Darstellung einer Beschichtung, bei der die Organosiliziumverbindung ein Polysiloxan ist.

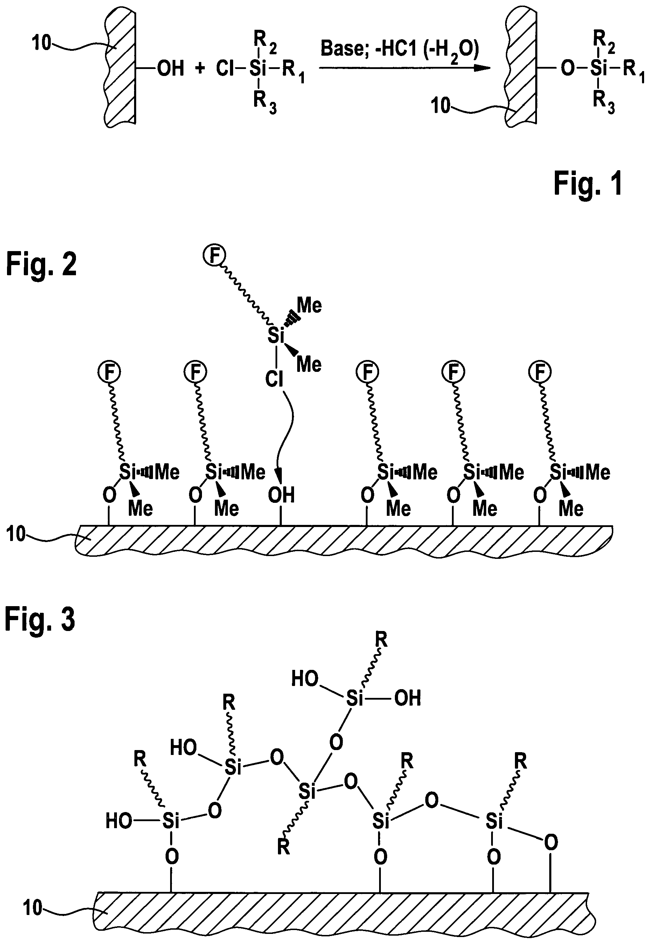

Fig. 1 dient der Illustration der Vorgänge bei einer Beschichtung einer Implantatsoberfläche 10 aus einem biokorrodierbaren metallischen Werkstoff. Die Implantatsoberfläche 10 weist eine OH-Funktionalität auf. Durch Umsetzung mit dem dargestellten Chlorsilan unter wasserfreien basischen Bedingungen erfolgt dessen kovalente Anbindung an die Implantatsoberfläche 10. Die Reste R1 bis R3 des Chlorsilans werden wie zuvor genannt festgelegt.

Fig. 2 stellt schematisch die Abläufe während einer Funktionalisierung der Implantatsoberfläche 10 mit einem Silan dar, das - neben zwei Methylgruppen - einen langkettigen, unverzweigten Alkylrest mit einer terminal angeordneten reaktiven Gruppe (mit F bezeichnet) aufweist. Der langkettige Rest bildet eine hydrophobe Sperrschicht. Durch die langkettigen Alklyreste, welche eine homogene dichte Schicht ausbilden, bleibt die Funktion als korrosionshemmende Sperrschicht auch in Bereichen hoher mechanischer Verformung des Grundkörpers erhalten. Die Organosiliziumschicht adaptiert sich den gegebenen sterischen Randbedingungen, wobei durch die starke hydrophobe Kraft auf die parallel angeordneten Alkylreste eine geschlossene Schicht erhalten bleibt.

Fig. 3 ist eine Beschichtung aus einem kovalent gebundenen Polysiloxan zu entnehmen.

### Vergleichsbeispiel 1 - Stentbeschichtung mit 3-Aminopropyltriethoxysilan

Stents aus der biokorrodierbaren Magnesiumlegierung WE43 (93 Gew.% Magnesium, 4 Gew.% Yttrium (W) und 3 Gew.% Seltenerdmetalle (E) außer Yttrium) wurden mit Isopropanol unter Ultraschall gewaschen und getrocknet.

Es wurde eine Beschichtungslösung aus 18 ml wasserfreiem Toluol, 2.2 ml 3-Aminopropyltriethoxysilan und 1 ml Triethylamin angesetzt.

Die Stents wurden für 4 Stunden bei 75 °C in der Beschichtungslösung inkubiert, wieder entnommen, mit Toluol gewaschen und bei etwa 90 °C für eine Stunde im Vakuumofen getrocknet.

### Vergleichsbeispiel 2 - Stentbeschichtung mit 3-Mercapto-propyl-trimethoxysilan

Stents aus der biokorrodierbaren Magnesiumlegierung WE43 (93 Gew.% Magnesium, 4 Gew.% Yttrium (W) und 3 Gew.% Seltenerdmetalle (E) außer Yttrium) wurden mit Chloroform gewaschen und getrocknet.

Es wurde eine Beschichtungslösung aus 90 Gew.% Methanol, 6 Gew.% Wasser und 4 Gew.% 3-Mercapto-propyl-trimethoxysilan (PropS-SH) angesetzt. Der pH-Wert wird durch Zugabe von Essigsäure auf 4.5 - 5.5 eingestellt.

Die Stents wurden für 30 min. bei Raumtemperatur in die Beschichtungslösung getaucht, wieder entnommen, mit Methanol gewaschen und bei etwa 60 °C für eine Stunde im Vakuumofen getrocknet.

### Vergleichsbeispiel 3 - Stentbeschichtung mit n-Octadecyltrichlorsilan

Stents aus der biokorrodierbaren Magnesiumlegierung WE43 (93 Gew.% Magnesium, 4 Gew.% Yttrium (W) und 3 Gew.% Seltenerdmetalle (E) außer Yttrium) wurden mit Chloroform gewaschen und getrocknet.

Es wurde eine Beschichtungslösung aus 95 Gew.% Chlorbenzol, und 5 Gew.% n-Octadecyltrichlorsilan angesetzt.

Die Stents wurden unter getrocknetem Stickstoff für 5 min. bei Raumtemperatur in die Beschichtungslösung getaucht. Nach der Silanisierung wurden die Stents mit Chlorbenzol gewaschen, für 10 min. in Ethanol unter Ultraschall gereinigt und bei etwa 60 °C für eine Stunde im Vakuumofen getrocknet.

## Patentansprüche

1. Implantat aus einem biokorrodierbaren metallischen Werkstoff mit einer Beschichtung aus einer Organosiliziumverbindung der Formel (1): 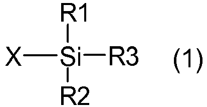 wobei X für eine O-, S- oder N-Funktionalität an einer Oberfläche des Implantats steht, über die eine kovalente Anbindung der Organosiliziumverbindung an die Oberfläche des Implantats erfolgt;
R1 und R2 unabhängig voneinander festgelegt ein substituierter oder unsubstituierter Alkylrest mit 1 bis 5 C-Atomen oder eine Sauerstoffbrücke zu einer benachbarten Organosiliziumverbindung ist; und
R3 ein substituierter oder unsubstituierter Alkylrest ist und der Alkylrest 3 bis 30 C-Atomen aufweist, wobei als Substituenten Halogenide, Aromaten oder heteroaromatische Verbindungen vorhanden sind, bei dem der biokorrodierbare metallische Werkstoff eine biokorrodierbare Legierung ausgewählt aus der Gruppe Magnesium, Eisen und Wolfram ist.

2. Implantat nach Anspruch 1, wobei der biokorrodierbare metallische Werkstoff eine biokorrodierbare Legierung ist, deren Hauptkomponente Magnesium, Eisen oder Wolfram ist.

3. Implantat nach Anspruch 1 oder 2, bei dem der biokorrodierbare metallische Werkstoff eine Magnesiumlegierung ist.

4. Implantat nach Anspruch 1, wobei das Implantat ausgewählt ist aus der Gruppe bestehend aus Befestigungselemente für Knochen, chirurgisches Nahtmaterial, Darmklammern, Gefäßclips, Ankerelemente für Elektroden und Stents, und vorzugsweise ein Stent ist.

5. Implantat nach Anspruch 1, bei dem R1 und R2 unabhängig voneinander festgelegt einem Substituenten ausgewählt aus der Gruppe Methyl, Ethyl, n-Propyl und i-Propyl entspricht.

6. Implantat nach einem der vorhergehenden Ansprüche, bei dem R1 und R2 unabhängig voneinander festgelegt ein substituierter oder unsubstituierter Alkylrest mit 1 bis 5 C-Atomen ist.

7. Implantat nach nach einem der vorhergehenden Ansprüche, bei dem R3 ein substituierter Alkylrest ist und der Substituent Chlor ist.

8. Implantat nach Anspruch 1, bei dem R3 terminal einen reaktiven Substituenten trägt.

9. Verfahren zur Herstellung eines Implantats aus einem biokorrodierbaren metallischen Werkstoff mit einer Beschichtung aus einer Organosiliziumverbindung der Formel (1): 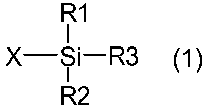 wobei X für eine O-, S- oder N-Funktionalität an einer Oberfläche des Implantats steht, über die eine kovalente Anbindung der Organosiliziumverbindung an die Oberfläche des Implantats erfolgt;
R1 und R2 unabhängig voneinander festgelegt ein substituierter oder unsubstituierter Alkylrest mit 1 bis 5 C-Atomen oder eine Sauerstoffbrücke zu einer benachbarten Organosiliziumverbindung ist; und
R3 ein substituierter oder unsubstituierter Alkylrest oder eine Alkylbrücke zu einer benachbarten Organosiliziumverbindung ist und der Alkylrest/die Alkylbrücke 3 bis 30 C-Atomen aufweist, wobei 1 bis 3 C-Atome durch ein Heteroatom ausgewählt aus der Gruppe O, N und S ersetzbar sind;
und wobei das Verfahren die Schritte umfasst:
(i) Bereitstellen eines Rohlings für das Implantats bestehend aus dem biokorrodierbaren metallischen Werkstoff;
(ii) Vorbehandeln einer Rohlingsoberfläche zur Erzeugung von O- S- oder N-Funktionalitäten durch Plasmabehandlung in sauerstoff- oder stickstoffreicher Atmosphäre; und
(iii) Beschichten der Rohlingsoberfläche mit einem Organosiliziumreagenz, das unter Ausbildung einer kovalenten Bindung zwischen Silizium und einer O-, S- oder N-Funktionalität abreagiert, wobei sich entweder die Organosiliziumverbindung der Formel (1) direkt bildet oder zunächst eine Vorläufer-Organosiliziumverbindung anfällt, die über weitere Behandlungsschritte in die Organosiliziumverbindung der Formel (1) überführt wird.

## Claims

1. An implant made of a biocorrodible metallic material having a coating made of an organosilicon compound of formula (1): 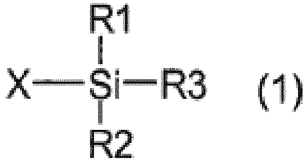 wherein X signifies an O, S, or N functionality on a surface of the implant, via which the organosilicon compound covalently bonds to the surface of the implant;
R1 and R2, established independently of one another, are a substituted or unsubstituted alkyl group having 1 to 5 C atoms or an oxygen bridge to a neighbouring organosilicon compound; and
R3 is a substituted or unsubstituted alkyl group and the alkyl group has 3 to 30 C atoms, wherein halides, aromatics or heteroaromatic compounds are provided as substituents,
wherein the biocorrodible metallic material is a biorcorrodible alloy selected from the group of magnesium, iron and tungsten.

2. The implant according to claim 1, wherein the biocorrodible metallic material is a biocorrodible alloy of which the main component is magnesium, iron, or tungsten.

3. The implant according to claim 1 or 2, wherein the biocorrodible metallic material is a magnesium alloy.

4. The implant according to claim 1, wherein the implant is selected from the group consisting of fasteners for bones, surgical suture material, intestinal clamps, vascular clips, anchoring elements for electrodes, and stents, and is preferably a stent.

5. The implant according to claim 1, wherein R1 and R2, established independently of one another, correspond to a substituent selected from the group consisting of methyl, ethyl, n-propyl, and i-propyl.

6. The implant of any one of the preceding claims, wherein R1 and R2, established independently of one another, are a substituted or unsubstituted alkyl group having 1 to 5 C atoms.

7. The implant of any one of the preceding claims, wherein R3 is a substituted alky group and the substituent is chlorine.

8. The implant according to claim 1, wherein R3 carries a reactive substituent terminally.

9. A method for producing an implant made of a biocorrodible metallic material having a coating made of an organosilicon compound of formula (1): 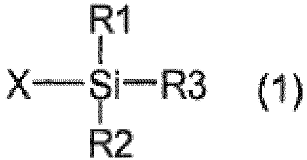 wherein X signifies an O, S, or N functionality on a surface of the implant, via which the organosilicon compound covalently bonds to the surface of the implant;
R1 and R2, established independently of one another, are a substituted or unsubstituted alkyl group having 1 to 5 C atoms or an oxygen bridge to a neighbouring organosilicon compound; and
R3 is a substituted or unsubstituted alkyl group or an alkyl bridge to a neighbouring organosilicon compound and the alkyl group/the alkyl bridge has 3 to 30 C atoms, wherein 1 to 3 C atoms can be replaced by a heteroatom selected from the group O, NandS:
and wherein the method comprises the following steps:
(i) providing a blank for the implant made of the biocorrodible metallic material;
(ii) pretreating a blank surface to generate O, S, or N functionalities by plasma treatment in oxygen-rich or nitrogen-rich atmosphere; and
(iii) coating the blank surface using an organosilicon reagent, which reacts between silicon and an O, S, or N functionality to form a covalent bond, wherein either the organosilicon compound of formula (1) forms directly, or first a precursor organosilicon compound occurs, which is converted via further treatment steps into the organosilicon compound of the formula (1).

## Revendications

1. Implant à base d'un matériau métallique biocorrodable doté d'un revêtement à base d'un composé d'organosilicium selon la formule (1) : 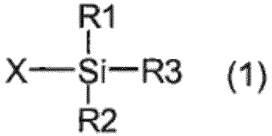 où X représente une fonctionnalité O-, S- ou N- à la surface de l'implant, par laquelle une liaison covalente du composé d'organosilicium est réalisée à la surface de l'implant ;
R1 et R2, définis indépendamment l'un par rapport à l'autre, sont un radical alkyle substitué ou non substitué, avec 1 à 5 atomes de C et un pont d'oxygène vers un composé d'organosilicium voisin ; et
R3 est un radical alkyle substitué ou non substitué et le radical alkyle présente de 3 à 30 atomes de C, où, en tant que substituants, il y a des composés halogénés, aromatiques ou hétéro aromatiques, chez lequel le matériau métallique biocorrodable est un alliage biocorrodable choisi dans le groupe du magnésium, du fer et du tungstène.

2. Implant selon la revendication 1, dans lequel le matériau métallique biocorrodable est un alliage biocorrodable dont le composant principal est du magnésium, du fer ou du tungstène.

3. Implant selon la revendication 1 ou 2, chez lequel le matériau métallique biocorrodable est un alliage de magnésium.

4. Implant selon la revendication 1, où l'implant est choisi dans le groupe constitué d'éléments de fixation pour des os, de matériel de suture chirurgical, d'agrafes abdominales, de clips pour les vaisseaux, d'éléments d'ancrage pour des électrodes et de stents, et est de préférence un stent.

5. Implant selon la revendication 1, chez lequel R1 et R2, définis indépendamment l'un par rapport à l'autre, sont un substituant choisi dans le groupe constitué des groupements méthyle, éthyle, n-propyle et i-propyle.

6. Implant selon l'une des revendications précédentes, chez lequel R1 et R2, définis indépendamment l'un par rapport à l'autre, sont un radical alkyle substitué ou non substitué avec 1 à 5 atomes de C.

7. Implant selon l'une des revendications précédentes, chez lequel R3 est un radical alkyle substitué et le substituant est du chlore.

8. Implant selon la revendication 1, chez lequel le radical R3 terminal porte un substituant réactif.

9. Procédé de fabrication d'un implant à base d'un matériau métallique biocorrodable avec un revêtement à base d'un composé d'organosilicium selon la formule (1) : 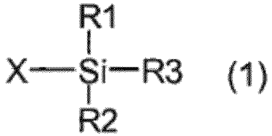 où X représente une fonctionnalité O-, S- ou N- à la surface de l'implant, par laquelle une liaison covalente du composé d'organosilicium est réalisée à la surface de l'implant ;
R1 et R2, définis indépendamment l'un par rapport l'autre, sont un radical alkyle substitué ou non substitué, avec 1 à 5 atomes de C et un pont d'oxygène vers un composé d'organosilicium voisin ; et
R3 est un radical alkyle substitué ou non substitué ou un pont alkyle vers un composé d'organosilicium voisin et le radical alkyle/le pont alkyle présente de 3 à 30 atomes de C, où 1 à 3 atomes de C peuvent être remplacés par un hétéroatome choisi dans le groupe O, N et S;
et où le procédé comprend les étapes :
(i) de mise à disposition d'une pièce brute pour l'implant constituée du matériau métallique biocorrodable ;
(ii) de prétraitement de la surface de la pièce brute pour la création de fonctionnalités O-, S- ou N- par un traitement par plasma dans une atmosphère d'oxygène ou d'azote ; et
(iii) de revêtement de la surface de la pièce brute avec un réactif d'organosilicium qui, moyennant la formation d'une liaison covalente entre le silicium et l'une des fonctionnalités O-, S- ou N-, cesse de réagir, où, soit le composé d'organosilicium selon la formule (1) se forme directement, soit un précurseur de composé d'organosilicium se dépose, qui est transformé en le composé d'organosilicium selon la formule (1) par d'autres étapes de traitement.
